(19) Europäisches Patentamt
European Patent Office
Office européen des brevets

(11) **EP 3 040 062 B1**

(12) **EUROPÄISCHE PATENTSCHRIFT**

(45) Veröffentlichungstag und Bekanntmachung des Hinweises auf die Patenterteilung:
**13.09.2017 Patentblatt 2017/37**

(51) Int Cl.:
***A61F 13/534*** *(2006.01)*     ***A61L 15/46*** *(2006.01)*
***A61F 13/84*** *(2006.01)*     ***A61L 15/28*** *(2006.01)*

(21) Anmeldenummer: **14200368.0**

(22) Anmeldetag: **29.12.2014**

(54) **Absorbierende Struktur und absorbierender Artikel enthaltend diese absorbierende Struktur**

Absorbent structure, and absorbent item comprising this absorbent structure

Structure absorbante et article absorbant contenant ladite structure absorbante

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**06.07.2016 Patentblatt 2016/27**

(73) Patentinhaber:
• **Paul Hartmann AG**
**89522 Heidenheim (DE)**
Benannte Vertragsstaaten:
**AL AT BE BG CH CY CZ DE DK EE ES FI FR GB GR HR HU IE IS LI LT LU LV MC MK MT NL NO PL PT RO RS SE SI SK SM**
• **Glatfelter Falkenhagen GmbH**
**16928 Pritzwalk (DE)**
Benannte Vertragsstaaten:
**AL AT BG CH CY CZ DE DK EE FI FR GR HR HU IE IS IT LI LT LU LV MC MK MT NL NO PT RO RS SE SI SK SM TR**

(72) Erfinder:
• **Ebert, Anselm**
**89522 Heidenheim (DE)**
• **Eilers, Jörg**
**89073 Ulm (DE)**
• **Lutter, Stefanie**
**16928 Pritzwalk (DE)**
• **Röttger, Henning**
**16928 Pritzwalk (DE)**

(74) Vertreter: **Oltmann, Eckhard et al**
**Paul Hartmann AG**
**Patents & Licensing SM-PL**
**Paul-Hartmann-Straße 12**
**89522 Heidenheim (DE)**

(56) Entgegenhaltungen:
**EP-A2- 2 184 042**     **EP-B1- 0 991 436**
**WO-A1-00/35502**     **WO-A1-2008/058563**
**DE-A1-102010 006 228**     **US-A1- 2002 069 988**

EP 3 040 062 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

**Beschreibung**

Die vorliegende Erfindung betrifft eine absorbierende Struktur mit einer Abfolge an miteinander verbundenen Lagen umfassend eine erste äußere absorbierende Lage aus Airlaid-Material, eine zweite äußere absorbierende Lage aus Airlaid-Material und eine zwischen der ersten und zweiten äußeren absorbierenden Lage angeordnete Flüssigkeitsspeicherlage aus Airlaid-Material.

Absorbierende Strukturen finden Anwendung in absorbierenden Artikeln insbesondere im Bereich der Hygiene wie beispielsweise in Kinderwindeln, Damenhygiene-Produkten oder Inkontinenz-Produkten sowie im Bereich der Wundabdeckung und Wundbehandlung, wie beispielsweise in Wundauflagen.

Absorbierende Strukturen aus einem Verbund an aufeinanderfolgenden Lagen aus Airlaid-Material sind bekannt.

So zeigt DE 10 2009 055 951 A1 eine mindestens aus drei Lagen bestehende absorbierende Struktur aus einer Flüssigkeitsaufnahmeschicht, einer nachgeschalteten Flüssigkeitsspeicherschicht beinhaltend SAP und einer Flüssigkeitsverteilungsschicht, dabei bestehen vorzugsweise alle Schichten aus einem Airlaid-Material. Weitere Beispiele für eine derartige Lagenabfolge an Airlaid-Materialien sind aus DE 10 2010 006 228 A1 und EP 1 191 915 B1 bekannt.

Absorbierende Strukturen sind unabhängig von ihrem Aufbau oftmals mit dem Problem behaftet, dass die darin angesammelte Flüssigkeit, nämlich Körperflüssigkeit mit organischen Komponenten, einen Nährboden für das Wachstum von Bakterien und sonstigen Mikroorganismen darstellt. Hautprobleme durch Hautreizungen und auch Geruchsentwicklung sind oftmals die Folge.

Diesem grundsätzlichen Problem entgegentretend ist die Einstellung von sauren pH-Werten in Faseransammlungen durch Zusatz von sauren, pH-Wert kontrollierenden Substanzen oder Zusatz von sauren Fasern bekannt:

So zeigt EP 0 138 179 A2 die Herstellung von sauren Fasern und deren Einfluss zur Neutralisierung eines basischen Testserums.

EP 0 202 127 B1 offenbart absorbierende Hygieneartikel mit einer Anordnung an separaten Lagen von pH-Wert kontrollierenden Komponenten und SAP.

EP 0 991 436 B1 zeigt absorbierende Artikel mit im Saugkörper enthaltenen pH-Wert kontrollierenden Substanzen in Form eines teilweise pH-neutralisierten SAPs und Fluffpulps mit einem pH-Wert unter 7. Bei Einnässen des Artikels pendelt sich der pH-Wert im Produkt zwischen 3,5 und 4,9 ein, was u.a. zur Erniedrigung von Enzymaktivitäten vorhandener Mikroorganismen führt.

Weiter sind aus EP 2 086 596 B1 absorbierende Artikel typischen Aufbaus mit einem Saugkörper bekannt, wobei der Saugkörper saure Cellulosefasern mit einem pH-Wert kleiner/gleich 5,5 und ein organisches Zinksalz zur Unterdrückung oder Reduktion der Ammoniakproduktion aufweist. Ähnliches ist der WO 2008/138386 A1 zu entnehmen, welche absorbierende Artikel mit Saugkörper umfassend saure Cellulosefasern oder sauren SAP mit einem pH-Wert kleiner/gleich 5,5 und einem Zusatz von Benzoesäure, Hydroxybenzoesäure oder einen Ester zeigt.

Der vorliegenden Erfindung liegt die Aufgabe zu Grunde, eine absorbierende Struktur oder eine solche absorbierende Struktur beinhaltende absorbierende Artikel bereitzustellen, welche sowohl absorbierende Eigenschaften und als auch Mikroorganismenwachstum reduzierende oder hemmende Eigenschaften aufweisen und dem Anwender der absorbierenden Struktur dabei ein höheres Maß an Anwendungskomfort gewährleisten. Zudem sollte die absorbierende Struktur in ihrer Anwendung flexibel angeordnet werden können, und so auch prozesstechnisch einfach und flexibel in einen laufenden Herstellungsprozess eines absorbierenden Artikels eingebracht werden können.

Diese Aufgabe wird erfindungsgemäß gelöst durch eine absorbierende Struktur mit einer Abfolge an miteinander verbundenen Lagen umfassend eine erste äußere absorbierende Lage aus Airlaid-Material, eine zweite äußere absorbierende Lage aus Airlaid-Material und eine zwischen der ersten und zweiten äußeren absorbierenden Lage angeordnete Flüssigkeitsspeicherlage aus Airlaid-Material, wobei das Airlaid-Material der ersten absorbierenden Lage erste Cellulose-Fasern, das Airlaid-Material der zweiten äußeren absorbierenden Lage zweite Cellulose-Fasern und das Airlaid-Material der Flüssigkeitsspeicherlage dritte Cellulose-Fasern und superabsorbierende Komponenten aufweisen, wobei die ersten und die zweiten Cellulose-Fasern einen geringeren pH-Wert aufweisen als die dritten Cellulose-Fasern und wobei der pH-Wert der ersten und der zweiten Cellulose-Fasern kleiner 5,0 ist. Der pH-Wert der ersten und der zweiten Cellulose-Fasern und auch der pH-Wert der dritten Cellulose-Fasern werden nach der in der Beschreibung dargelegten Methode bestimmt.

Mit dieser Erfindung wurde erkannt, dass ein Airlaid-Material im Vergleich zu anderen Vliestypen, wie Meltblown-Vlies oder Spinnvlies, ein Kavitäten aufweisendes, also Zwischenräume bildendes Fasermaterial ist und dass dessen Ausstattung mit sauren Cellulose-Fasern mit einem pH-Wert kleiner 5,0 eine optimale Kombination für das Auffangen und Speichern von Flüssigkeiten in einer Mikroorganismenwachstum reduzierenden oder hemmenden Umgebung darstellt. Wenngleich ein saurer pH-Wert zur Reduktion oder Hemmung von Mikroorganismen-Wachstum gewollt ist, ist eine ungehinderte Freisetzung von sauren Agenzien in derartig hautnahen Anwendungsbereichen von Hygiene- und Wundversorgungsprodukten nicht unbedingt wünschenswert. Diese sauren Cellulose-Fasern im Airlaid-Material können im Gegensatz zu einer sauren Ausstattung von Faseranhäufungen mittels Zusatz von ungebundenen pH-Kontrollmitteln, wie Säuren oder Salzen von Säuren, direkt als ein saures Biopolymer agieren. Ein Airlaid-Material mit sauren Cellulose-Fasern kann ohne großes Auswaschen der sauren Komponente eine permanente Ummantelung der im Airlaid-Material aufgenommenen und/oder gespeicherten Flüssigkeit bereitstellen.

Dadurch, dass das Airlaid-Material sowohl der ersten als auch der zweiten äußeren absorbierenden Lage erste bzw. zweite Cellulose-Fasern mit einem pH-Wert kleiner 5,0 aufweist, lassen sich diese erfindungsgemäßen absorbierenden Strukturen flexibel einsetzen. Sowohl die erste als auch die zweite äußere absorbierende Lage kann als die körperzugewandte Lage bei der Anwendung der absorbierenden Struktur oder bei der Anordnung der absorbierenden Struktur innerhalb eines absorbierenden Artikels eingesetzt werden. Unabhängig von der Orientierung der absorbierenden Struktur liegt somit stets eine zum Anwender gerichtete und mit sauren pH-Wert Bedingungen ausgestattete Lage vor. Zusätzlich kann die andere der beiden äußeren absorbierenden Lagen, welche die körperabgewandte und in der Ausführung eines absorbierenden Hygieneartikels somit die kleidungszugewandte Lage darstellt, bei möglicherweise fehlerhaft außerhalb der absorbierenden Struktur in Richtung Kleidungsseite gewanderter Flüssigkeit einen weiteren Aufnahme-, Verteilungs- oder Speicherort mit Mikroorganismenwachstum hemmenden Bedingungen bereitstellen. Die Quellfähigkeit von SAP ist durch die Ionenstärke und den pH-Wert der Umgebung bestimmt, so dass ein nicht zu saurer pH-Wert der dritten Cellulose-Fasern im Airlaid-Material der Flüssigkeitsspeicherlage positiv zur Speicherkapazität des SAP und damit auch zur Absorptionskapazität der absorbierenden Struktur beiträgt.

Airlaid-Material wird hierbei verstanden als eine Faseranhäufung von trocken abgelegten Stapelfasern, also von Fasern mit einer endlichen oder definierten, vorzugsweise geschnittenen Länge. Stapelfasern können dabei Naturfasern, wie beispielsweise Baumwolle, Fluffpulp, Hanf, oder synthetische Stapelfasern mit definierter oder geschnittener Länge basierend auf Naturfasern, wie beispielsweise Viskose oder basierend auf Polymeren, wie Polyolefinen, Polyamiden, Polyestern oder Kombinationen aus Polymeren sein. Bei einem Meltblown-Material oder Spunbond-Material werden dagegen die frisch extrudierten Meltblown-Fasern oder Spunbond-Fasern mit endloser Länge ihrem Herstellungsprozess direkt nachgeschaltet und zumeist noch im klebrigen Zustand zu einer Faseranhäufung abgelegt. Das Airlaid-Material der ersten äußeren absorbierenden Lage, der zweiten äußeren absorbierenden Lage und der Flüssigkeitsspeicherlage enthält somit keine Meltblown-Fasern oder Spundbond-Fasern. Dem Airlaid-Material können dabei durchaus weitere Komponenten, wie andere Stapelfasern oder superabsorbierende Komponenten in Faser- oder Partikelform beigemengt sein.

Die Bereitstellung von Lagen aus Airlaid-Material ist dem Fachmann bekannt. Die in einem separaten vorgeschalteten Prozess hergestellten synthetischen Stapelfasern oder die Naturfasern werden dazu in einem Luftlegeverfahren abgelegt. Werden unterschiedliche Stapelfasern im Airlaid-Material verwendet, so werden diese vorzugsweise in einem Luftstrom zuvor vermischt und dann nach üblichen Vliesbildungsverfahren abgelegt. Das Airlaid-Material als solches kann weiteren fachüblichen Weiterbearbeitungsmaßnahmen, wie Kardieren oder Verfestigen, wie beispielsweise Kalandrieren oder Thermofixierung, unterworfen werden.

In der erfindungsgemäßen absorbierenden Struktur sind in der ersten und zweiten äußeren absorbierenden Lage vorzugsweise jeweils mehr erste Cellulose-Fasern respektive zweite Cellulose-Fasern als dritte Cellulose-Fasern und in der Flüssigkeitsspeicherlage mehr dritte Cellulose-Fasern als erste und/oder zweite Cellulose-Fasern enthalten. Weiter vorzugsweise bestehen wenigstens 70 Gew-% des Fasermaterials des jeweiligen Airlaid-Materials der ersten respektive der zweiten äußeren absorbierenden Lage aus ersten Cellulose-Fasern respektive zweiten Cellulose-Fasern und wenigstens 70 Gew-% des Fasermaterials des Airlaid-Materials der Flüssigkeitsspeicherlage aus dritten Cellulose-Fasern.

Insbesondere bevorzugt ist eine absorbierende Struktur, bei der das Airlaid-Material der ersten und/oder zweiten äußeren absorbierenden Lage keine dritten Cellulose-Fasern aufweist. Insbesondere bevorzugt sind im Airlaid-Material beider äußeren absorbierenden Lagen keine dritten Cellulose-Fasern enthalten. Insbesondere bevorzugt ist eine absorbierende Struktur, bei der das Airlaid-Material der Flüssigkeitsspeicherlage keine ersten oder zweiten Cellulose-Fasern aufweist. Zu dem vorstehend Gesagten wird das Verständnis zugrunde gelegt, dass dem Airlaid-Material der jeweiligen Lage aus ersten oder zweiten Cellulose-Fasern bzw. dritten Cellulose-Fasern keine der anderen dritten bzw. zweiten oder ersten Cellulose-Fasern bewusst beigemengt oder beigemischt sind. Dabei ist aber nicht ausgeschlossen, dass in den Grenzbereichen zwischen den einzelnen jeweiligen Lagen einzelne Fasern der jeweiligen Lage durchaus in die benachbarte Lage hineinragen können. Dies kann insbesondere bei einem inline-Herstellungsprozess durch direktes geschichtetes Ablegen von Airlaid-Materiallagen bedingt sein.

In einer vorteilhaften Ausführung der absorbierenden Struktur weisen die ersten und/oder die zweiten Cellulose-Fasern einen pH-Wert kleiner 4,7, vorzugsweise kleiner 4,5, vorzugsweise kleiner 4,2, und weiter vorzugsweise einen pH-Wert größer 3,2, weiter vorzugsweise größer 3,5, weiter vorzugsweise größer 3,7, weiter vorzugsweise größer 3,9 auf.

Um den Aspekt der vorteilhaften Wirkungen von saurem pH zur Reduktion oder Hemmung des Wachstums von Mikroorganismen und/oder auch Verminderung oder Hemmung von Geruchsentwicklungen fortzuführen, weisen insbesondere auch die dritten Cellulose-Fasern in der Flüssigkeitsspeicherlage einen pH-Wert kleiner pH 7 auf. Insbesondere bevorzugt weisen die dritten Cellulose-Fasern einen pH kleiner 6,7, weiter bevorzugt kleiner 6,5, weiter bevorzugt kleiner 6,3, und weiter bevorzugt größer pH 5,0, weiter bevorzugt größer 5,5, weiter bevorzugt größer 5,7, weiter bevorzugt größer 5,9 auf.

In einer bevorzugten Ausführungsform ist die absorbierende Struktur derart ausgestaltet, dass der pH-Wert der ersten und/oder der zweiten Cellulose-Fasern sich von dem pH-Wert der dritten Cellulose-Fasern um mindestens 0,5, bevorzugt mindestens 0,7, weiter bevorzugt mindestens 0,9, weiter bevorzugt mindestens 1,0, weiter bevorzugt höchstens 3,0, weiter bevorzugt höchstens 2,5 , weiter bevorzugt höchstens 2,0 unterscheidet.

Vorzugsweise sind in der absorbierenden Struktur die ersten und die zweiten Cellulose-Fasern der ersten und zweiten äußeren absorbierenden Lage in Summe in einem Anteil von 20 - 50 Gew-%, bevorzugt von 25 - 45 Gew-%, weiter bevorzugt von 30 - 40 Gew-% bezogen auf das Gesamtgewicht der Abfolge an miteinander verbundenen Lagen enthalten.

Als erste, zweite und dritte Cellulose-Fasern können Fasern jeglichen Ursprungs eingesetzt werden, solange Cellulose enthalten ist, wie bei Naturfasern, z.B. Bambusfasern, Baumwollfasern oder Holz und daraus gewonnenes Fluffpulp, oder insoweit Cellulose als Ausgangsstoff für die Weiterverarbeitung oder Aufbereitung zu daraus gewonnenen Regenerat-Fasern wie beispielsweise Viskosefasern dient. Insbesondere bevorzugt enthalten die ersten und/oder zweiten Cellulose-Fasern und/oder die dritten Cellulose-Fasern cellulosisches Fasermaterial aus der Gruppe Baumwollfasern und/oder Fluffpulp. Weiter vorzugsweise bestehen die ersten, zweiten und/oder dritten Cellulose-Fasern aus cellulosischem Fasermaterial aus der Gruppe Baumwollfasern und/oder Fluffpulp. Insbesondere bevorzugt können für die ersten und zweiten Cellulose-Fasern die identischen Fasern eingesetzt werden. Insbesondere bevorzugt werden sowohl für die ersten und zweiten als auch für die dritten Cellulose-Fasern Fluffpulp-Fasern eingesetzt, die sich erfindungsgemäß in ihren pH-Werten unterscheiden. Erste und/oder zweite Cellulose-Fasern mit einem pH-Wert kleiner pH 5,0 auf der Basis von Fluffpulp können beispielsweise von der Firma Weyerhaeuser, Washington, USA bezogen werden.

Die ersten und/oder zweiten Cellulose-Fasern mit einem pH-Wert kleiner pH 5,0 sind vorzugsweise Reaktionsprodukte aus cellulosischem Fasermaterial, wie insbesondere Baumwollfasern oder Fluffpulp, mit Polycarbonsäuren oder Salzen davon und/oder Polyacrylsäuren oder Salzen davon. Saure Cellulose-Fasern können beispielsweise nach dem in EP 0 832 320 B1 oder in US 6,852,904 B2 aufgezeigten Verfahren hergestellt werden. Die für den Reaktionsprozess eingesetzten Polycarbonsäuren oder Salze davon können insbesondere der Gruppe Maleinsäure, Weinsäure, Zitronensäure, 1,2,3-Propan-Tricarbonsäure, 1,2,3,4-Butan-Tetracarbonsäure und deren Salze davon entnommen sein.

Das Airlaid-Material der ersten und/oder zweiten äußeren absorbierenden Lage weist vorzugsweise keine superabsorbierenden Komponenten auf. Die Vermeidung von superabsorbierenden Komponenten in den äußeren Lagen ist vorteilhaft um der Gefahr von möglichem Blocking direkt an den äußeren Schichten vorzubeugen. Die superabsorbierenden Komponenten werden vorzugsweise in der Flüssigkeitsspeicherlage eingebracht, um die Flüssigkeit im Wesentlichen dort durch die superabsorbierenden Komponenten zu immobilisieren. Die superabsorbierenden Komponenten der Flüssigkeitsspeicherschicht sind vorzugsweise in einem Anteil von mindestens 25 Gew-%, bevorzugt von mindestens 30 Gew-%, bevorzugt von mindestens 35 Gew-%, weiter bevorzugt von mindestens 40 Gew-%, weiter bevorzugt von höchstens 70 Gew-%, weiter bevorzugt von höchstens 60 Gew-%, weiter bevorzugt von höchstens 50 Gew-% bezogen auf das Gesamtgewicht der Abfolge an miteinander verbundenen Lagen enthalten. Die superabsorbierenden Komponenten (8) liegen vorzugweise in Form von SAP-Partikeln und/oder SAP-Fasern vor, insbesondere auf Basis oberflächenvernetzter Polyacrylate, die weiter vorzugsweise teilneutralisiert sind.

In einer vorteilhaften Weiterbildung ist die absorbierende Struktur mit Bindemitteln ausgestattet. So weist insbesondere das Airlaid-Material der ersten und/oder zweiten äußeren absorbierenden Lage Bindemittel, bevorzugt Bindefasern, weiter bevorzugt Bindefasern in Form von Bi- und/oder Mehrkomponentenfasern, auf. Insbesondere können die Bindefasern thermoplastische Materialien, wie insbesondere Polyester und/oder Polyolefine umfassen. Vorteilhaft werden Bikomponentenfasern mit Komponenten unterschiedlicher Schmelzpunkte eingesetzt. Insbesondere vorteilhaft können Bikomponentenfasern aus Polyethylenterephthalat und Polyethylen eingesetzt werden. Thermoplastische Fasern in den äußeren absorbierenden Lagen tragen vorteilhaft zum Weiterleiten der Flüssigkeit in die Flüssigkeitsspeicherlage bei. Dem folgend weist insbesondere das Airlaid-Material der Flüssigkeitsspeicherlage keine Bindefasern, bevorzugt keine Bindemittel auf.

Vorzugsweise sind im Airlaid-Material der ersten äußeren und/oder der zweiten äußeren absorbierenden Lage Bindefasern in Summe in einem Anteil von 2 - 10 Gew-%, bevorzugt von 2 - 7 Gew-%, weiter bevorzugt von 2 - 6 Gew-%, weiter bevorzugt 3-6 Gew-%, weiter bevorzugt 4 - 6 Gew-% bezogen auf das Gesamtgewicht der Abfolge an miteinander verbundenen Lagen enthalten.

Die Abfolge an miteinander verbundenen Lagen weist eine erste und eine zweite äußere Oberseite auf. In einer bevorzugten Weiterbildung der absorbierenden Struktur weist die erste und/oder zweite äußere Oberseite eine Bindemittelbeschichtung auf. Insbesondere kann die Bindemittelbeschichtung auf Basis eines dispergierten Polymers sein, insbesondere eines Ethylen-Vinylacetat-Copolymers. Eine Bindemittelbeschichtung auf der Oberseite der ersten und/oder zweiten äußeren absorbierenden Lage kann durch die Fasereinbindung durchaus vorteilhaft die Kapillarwirkung und Transferfunktion der äußeren Lagen, insbesondere bei Vorhandensein von Bindefasern in der ersten und/oder zweiten äußeren Lage, unterstützen. Auch trägt eine Bindemittelbeschichtung vorteilhaft zu einer Abriebfestigkeit der Oberseiten bei, was u.a. positiv bei der direkten Anwendung als auch bei einer nachgeschalteten Konfektionierung eines absorbierenden Artikels in einem maschinellen Herstellungsprozess ist.

In einer bevorzugten Ausgestaltung des Erfindungsgedankens, eine in der Anwendung oder im Herstellungsprozess eines absorbierenden Hygieneartikels möglichst flexibel anordenbare absorbierende Struktur bereitzustellen, weisen die erste und die zweite absorbierende Lage identische Eigenschaften auf. Vorzugsweise sind die erste und zweite äußere absorbierende Lage zumindest hinsichtlich einer Eigenschaft identisch, wobei die Eigenschaft entnommen ist aus der Gruppe enthaltend Flächengewicht, pH-Wert der ersten oder zweiten Cellulose-Fasern, chemische Zusammensetzung oder natürliche Herkunft oder Ursprung der ersten und zweiten Cellulose-Fasern, Gewichtsanteil an ersten oder zweiten Cellulose-Fasern, Gewichtsanteil an Bindefasern und eingesetzte Fasermischung des Airlaid-Materials.

Insbesondere bevorzugt sind die erste und die zweite äußere absorbierende Lage zumindest hinsichtlich des pH-Werts der ersten und zweiten Cellulose-Fasern identisch.

Insbesondere bevorzugt sind die erste und die zweite äußere absorbierende Lage zumindest hinsichtlich der chemischen Zusammensetzung oder der natürlichen Herkunft oder des Ursprungs der ersten Cellulose-Fasern und zweiten Cellulose-Fasern identisch.

Weiter bevorzugt sind die erste und zweite äußere absorbierende Lage hinsichtlich pH-Wert der ersten und der zweiten Cellulose-Fasern und hinsichtlich der chemischen Zusammensetzung oder der natürlichen Herkunft oder des Ursprungs der ersten und zweiten Cellulose-Fasern identisch.

Weiter bevorzugt sind die erste und die zweite äußere absorbierende Lage hinsichtlich pH-Wert der ersten und zweiten Cellulose-Fasern und Gewichtsanteil an ersten und zweiten Cellulose-Fasern identisch.

Insbesondere bevorzugt sind die erste und die zweite äußere absorbierende Lage hinsichtlich pH-Wert der ersten und zweiten Cellulose-Fasern, in der chemischen Zusammensetzung oder in der natürlichen Herkunft oder des Ursprungs der ersten und zweiten Cellulose-Fasern und hinsichtlich Gewichtsanteil an ersten und zweiten Cellulose-Fasern identisch.

In einer insbesondere bevorzugten Ausführungsform sind die erste und die zweite äußere absorbierende Lage hinsichtlich aller Eigenschaften identisch.

In einer weiter bevorzugten Weiterbildung zum Erhalt einer flexibel anordenbaren absorbierenden Struktur ist die Abfolge an miteinander verbundenen Lagen spiegelsymmetrisch, d.h. bei einem imaginären Querschnitt durch die Abfolge an miteinander verbundenen Lagen können die Lagen durch die Achsenspiegelung an ihrer Symmetrieachse auf sich selbst abgebildet werden. Es sind sowohl in Richtung der einen als auch der anderen Oberseite der Abfolge an miteinander verbunden Lagen die gleiche Anzahl an Lagen vorhanden, insbesondere stimmen diese Lagen in ihrer funktionalen Ausstattung, weiter insbesondere zumindest in einer Eigenschaft entnommen aus der Gruppe enthaltend Flächengewicht, pH-Wert der ersten oder zweiten Cellulose-Fasern, Gewichtsanteil an ersten oder zweiten Cellulose-Fasern, chemische Zusammensetzung oder natürliche Herkunft oder Ursprung der ersten und zweiten Cellulose-Fasern, Gewichtsanteil an Bindefasern und eingesetzte Fasermischung des Airlaid-Materials überein.

Insbesondere besteht die Abfolge an miteinander verbundenen Lagen aus der ersten und zweiten äußeren absorbierenden Lage und der dazwischen angeordneten Flüssigkeitsspeicherlage.

Die Abfolge an miteinander verbundenen Lagen kann durch das Zusammenfügen der in separaten Verfahrensschritten hergestellten ersten und zweiten absorbierenden Lagen und der Flüssigkeitsspeicherlage erhalten werden. So können die beispielsweise auf Hilfsträgermaterialien, wie beispielsweise Tissue oder Nonwoven, abgelegten oder auf entsprechenden Hilfsträgermaterialien fixierten Airlaid-Materiallagen mit Fixiermittel, wie Kleber, Ultraschall miteinander verbunden werden. Alternativ kann die Abfolge an miteinander verbundenen Lagen auch mittels eines inline-Verfahrens durch das übereinander Ablegen der einzelnen Lagen erhalten werden.

Eine Verfestigung der Abfolge an miteinander verbundenen Lagen kann zur Kapillarwirkung und damit auch Speicherwirkung der gesamten absorbierenden Struktur beitragen. Die Abfolge an miteinander verbundenen Lagen wird insbesondere mittels Druck und/oder mittels Temperatur, insbesondere bei Vorhandensein von Bindefasern in den ersten und/oder zweiten äußeren absorbierenden Lagen und/oder im Falle einer Bindemittelbeschichtung an den äußeren Oberseiten, verfestigt. Die Verfestigung erfolgt insbesondere ohne Bildung von Prägemustern, die womöglich, insbesondere bei absorbierenden Strukturen von geringer Dicke, die Gefahr von Bruchstellen in der absorbierenden Struktur darstellen können. Dazu erfolgt die Verfestigung vorzugsweise unter Einsatz von Glattwalzen.

Die absorbierende Struktur weist vorzugsweise eine Dicke von 1 - 6 mm, bevorzugt von 2 - 5 mm, weiter bevorzugt von 2 - 4 mm auf. Die Dicke wird dabei mit einem mechanischen Dickenmessgerät mit einer Messfläche von 25 cm$^2$ unter Einsatz eines Prüfdrucks von 5 g/cm$^2$ gemessen. Die Prüfmuster sind dabei an das Normklima von 23°C bei 50% relativer Luftfeuchte angeglichen. Absorbierende Strukturen mit derart geringen Dicken werden vorzugsweise in Damenhygienebinden, in Inkontinenzvorlagen oder auch in Wundauflagen eingesetzt.

Das Flächengewicht der absorbierenden Struktur beträgt vorzugsweise mindestens 300 g/m$^2$, weiter vorzugsweise mindestens 320 g/m$^2$, weiter vorzugsweise höchstens 500 g/m$^2$, weiter vorzugsweise höchstens 450 g/m$^2$, weiter vorzugsweise höchstens 400 g/m$^2$.

Insbesondere bevorzugt weist die absorbierende Struktur eine relative Absorptionskapazität von mindestens 18g/g, weiter bevorzugt von mindestens 20g/g, weiter bevorzugt von mindestens 22 g/g, weiter bevorzugt von höchstens 40 g/g, weiter bevorzugt von höchstens 30 g/g, weiter bevorzugt von höchstens 26 g/g, weiter bevorzugt von höchstens 24 g/g auf, dabei mit der Absorptionskapazität gemessen nach der nachfolgend beschriebenen Methode.

Insbesondere bevorzugt weist die absorbierende Struktur eine relative Retentionskapazität von mindestens 7 g/g, weiter bevorzugt von mindestens 9 g/g, weiter bevorzugt von mindestens 11 g/g, weiter bevorzugt von höchstens 18 g/g, weiter bevorzugt von höchstens 16 g/g, weiter bevorzugt von höchstens 14 g/g, weiter bevorzugt von höchstens 12 g/g auf, wobei die Retentionskapazität nach der nachfolgend beschriebenen Methode vermessen ist.

Die absorbierende Struktur kann in ihrer flächenhaften Erstreckung beliebig ausgebildet sein. Es sind rechteckige Formen, aber ebenso für den jeweiligen Einsatz angepasste Formen möglich.

Die erfindungsgemäße absorbierende Struktur in einer ihrer vorgenannten Ausbildungen kann als solche bereits einen absorbierenden Artikel bilden oder in einem absorbierenden Artikel enthalten sein.

Gegenstand der Erfindung ist auch ein absorbierender Artikel, welcher eine erfindungsgemäße absorbierende Struktur nach einer der vorangehend dargestellten Ausführungsformen enthält. Der absorbierende Artikel ist dabei vorzugsweise eine Inkontinenzvorlage, eine Damenhygienebinde, eine Inkontinenzwindel offenen Typs mit Verschlusssystemen, eine Inkontinenzwindel geschlossenen Typs, und/oder eine Wundauflage.

Für die Ausgestaltung des absorbierenden Artikels als Hygieneartikel, wie eine Damenhygienebinde, eine Inkontinenzvorlage oder eine Inkontinenzwindel ist an einer der beiden äußeren absorbierenden Lagen eine flüssigkeitsdurchlässige Lage und an der anderen der beiden äußeren absorbierenden Lagen eine flüssigkeitsundurchlässige Lage angeordnet. Insbesondere ist dabei vorgesehen, dass die flüssigkeitsdurchlässige Lage bei der Anwendung zum Körper des Anwenders gerichtet ist, während die flüssigkeitsundurchlässige Lage bei der Anwendung vom Körper des Anwenders abgewendet ist, und im Falle von absorbierenden Hygieneprodukten, zur Kleidung orientiert ist.

Flüssigkeitsdurchlässige und/oder insbesondere körperzugewandte Lagen umfassen ein Vlies oder bestehen vorzugsweise aus einem Vlies. Insbesondere vorteilhaft ist, wenn das Vlies ein Spunbondmaterial, ein Laminat aus Spunbondlagen (S) und Meltblownlagen (M) oder ein Stapelfaservliesmaterial oder Kombinationen davon umfasst. Insbesondere weisen diese Vliese ein Flächengewicht von mindestens 6 g/m$^2$, insbesondere von mindestens 10 g/m$^2$, insbesondere von höchstens 30 g/m$^2$, insbesondere von höchstens 20 g/m$^2$ auf.

Flüssigkeitsundurchlässige und/oder insbesondere körperabgewandte Lagen umfassen vorteilhafterweise thermoplastische Folien oder Filme, insbesondere eine mikroporöse Folie oder ein Vlies-Folien-Laminat mit einer der vorgenannten Folien- oder Filmschicht oder bestehen aus den vorgenannten Materialien.

Bei der Konfektionierung von Hygieneartikeln wird zwischen der absorbierenden Struktur und der flüssigkeitsdurchlässigen, insbesondere körperzugewandten Lage vorzugsweise noch eine Flüssigkeitsverteilerlage eingebracht. Die Flüssigkeitsverteilerlage kann vorzugsweise ein Vliesmaterial aus PP, PE oder Polyesterfasern sein.

Für die Ausgestaltung des absorbierenden Artikels als eine Wundauflage mit Fixierungsmöglichkeit auf der Haut des Anwenders ist vorzugsweise an eine der beiden äußeren absorbierenden Lagen ein sich über die Kontur der absorbierenden Struktur hinauserstreckendes Trägermaterial angeordnet und fixiert. Im Falle einer adhäsiven Wundauflage weist das Trägermaterial eine übliche kontinuierliche oder auch diskontinuierliche Beschichtung mit einem hautverträglichen Kleber auf. Die Trägermaterialien weisen vorzugsweise Eigenschaften, wie Atmungsaktivität, Wasserdampfdurchlässigkeit oder gute Ablösbarkeit des Klebers auf. Als Trägermaterialien können vorzugsweise flüssigkeitsdichte und wasserdampfdurchlässige Folien aus Polyester, Polyurethan, Polypropylen, Polyethylen oder Polyamid oder Mischungen davon eingesetzt werden. Ebenfalls können poröse Trägermaterialien aus Vliesmaterial oder textilem Material, wie beispielsweise Gewebe oder Gewirke eingesetzt werden.

Nachfolgend sind die verwendeten Messmethoden im Detail beschrieben:

Bestimmung des pH-Werts der ersten, zweiten und dritten Cellulose-Fasern

Die Bestimmung des pH-Wertes der ersten und zweiten Cellulose-Fasern ist an die DIN 53124 - August 1998 angelehnt.

An Prüfgeräten werden benötigt: Glas-Erlenmeyerkolben mit Stopfen, Präzisionswaage mit einer Ablesegenauigkeit von 0,01 g, Thermometer, pH-Glaselektrode mit einer Messgenauigkeit von pH= 0,05.

An Reagenzien werden benötigt: demineralisiertes Wasser mit einer Leitfähigkeit von kleiner 0,1 mS/m, Standard-Pufferlösungen (pH 7 und pH 4) zur Kalibrierung der pH-Elektrode.

Die pH-Wert-Bestimmung der jeweiligen Cellulose-Fasern erfolgt in der nach einer Kaltextraktion erhaltenen Lösung:

Für die Herstellung des wässrigen Extraktes wird eine Probenmenge an Cellulose-Fasermaterial von 2 g +/- 0,1 g mit 100 ml demineralisiertem Wasser mit einer Raumtemperatur von 23°C ± 2 °C bei 50% relativer Luftfeuchte eine Stunde lang in einem verschlossenen Glas-Erlenmeyerkolben extrahiert. Dazu wird das Probenmaterial zu Beginn und dann alle 15 min umgeschüttelt.

Bei der Probenvorbereitung ist folgendes zu beachten: Das für die Extraktion eingesetzte demineralisierte Wasser muss eine Leitfähigkeit von kleiner 0,1 mS/m aufweisen. Bei einer von 2,0 g abweichenden Einwaage an Cellulose-Fasermaterial ist direkt proportional das bereitgestellte Volumen an demineralisiertem Wasser von 100 ml entsprechend abweichend anzugleichen. Zudem ist bei der Einwaage an Cellulose-Fasermaterial der Trockengehalt der Cellulose-Fasern zu berücksichtigen. In Kenntnis des Trockengehaltes des Cellulose-Fasermaterials wird der Trockengehalt beim Einwiegen des Cellulose-Fasermaterials derart berücksichtigt, dass eine Einwaage von entsprechend 2,0 g Trockenmasse des Cellulose-Fasermaterials vorgenommen wird.

In dem bei der Kaltextraktion gewonnenen Extrakt wird der pH-Wert bei Normklima von 23°C $\pm$ 2 °C und 50% relativer Luftfeuchte gemessen. Vor der Bestimmung des pH-Wertes muss eine Kalibrierung der pH-Elektrode mit Standard-Pufferlösungen vorgenommen werden: Als pH-Elektrode wird eine pH-Einstabmesskette aus Glas mit dem Referenzsystem Ag/AgCl und als Referenzelektrolyt KCl 3mol/l, mit einer kegelförmigen Glasmembran mit einem pH-Einsatzbereich von 0 - 14 und dem Nullpunkt bei pH = 7,0 eingesetzt. Hierfür kann beispielsweise die pH-Elektrode BlueLine 14 pH von der Firma SI Analytics aus 55122 Mainz, Deutschland verwendet werden. Als Standard-Pufferlösungen können nach DIN 53124:1998-08, Anhang A hergestellte Standard-Pufferlösungen oder Handelsprodukte verwendet werden. Als Handelsprodukte können beispielsweise AVS TITRINORM® pH4 und AVS TITRINORM® pH7 von der Firma VWR International 64295 Darmstadt, Deutschland eingesetzt werden. Nach Kalibrieren und mehrmaligem Spülen der pH-Elektrode mit demineralisiertem Wasser wird die pH-Messung im Extrakt durchgeführt.

Die Anzahl der Prüfungen beträgt n = 3. Die Angabe erfolgt als Mittelwert aus diesen drei Messungen und dabei gerundet auf eine Dezimale.

Bestimmung des Trockengehaltes der ersten, zweiten und dritten Cellulose-Fasern:

Die Bestimmung des Trockengehalts (und damit der Trockenmasse) der ersten, zweiten und dritten Cellulose-Fasern bzw. der entsprechenden Cellulose-Fasern-Platte wird separat vor der pH-Wert-Messung durchgeführt.

Zur Probenvorbereitung wird im Falle von in Plattenform gepressten Cellulose-Fasern diese Cellulose-Faserplatte in Stücke gerissen oder geschnitten. Die Probenvorbereitung muss dabei zügig erfolgen, um Verluste an Feuchtegehalt auf ein Minimum zu reduzieren. Die Probenvorbereitung erfolgt im Normklima 23°C $\pm$ 2 °C / 50% relative Luftfeuchte.

An Prüfgeräten werden benötigt: ein wasserdampfdichtes Wägeglas mit dichtschließendem Deckel, ein Trockenschrank (105 $\pm$ 2 ° C), eine Präzisionswaage mit einer Ablesegenauigkeit von 0,0001 g und ein Exsikkator.

Für das Prüfverfahren wird das Wägeglas vorgetrocknet und zusammen mit dem Deckel gewogen (w1). Dann wird 5 g Cellulose-Faser-Material (m1) genau in das Wägeglas eingewogen. Nach dem Wiegen werden das Wägeglas mit dem Cellulose-Fasermaterial und der abgenommene Deckel bei einer konstanten Temperatur von 105 $\pm$ 2 °C in den Trockenschrank gestellt bis eine konstante Masse erreicht ist. Die Trockenperiode darf dabei nicht weniger als 3 h und auch nicht länger als 16 h sein. Die Masse des Prüfmusters gilt als konstant, wenn zwei aufeinanderfolgende Wägungen sich untereinander nicht mehr als 0,1 % der Ursprungsmasse des Prüfmusters unterscheiden. Bei Erreichen der Massenkonstanz wird das Wägeglas aus dem Trockenschrank genommen, der Deckel aufgesetzt und 45 Minuten zum Abkühlen in den Exsikkator gestellt. Nach dem Abkühlen wird das Wägeglas aus dem Exsikkator genommen und durch schnelles Halböffnen und Wiederschließen des Deckels wird der innere und äußere Luftdruck des Wägeglases ausgeglichen. Das Wägeglas mit seinem getrocknetem Inhalt und Deckel wird auf 0,0001 g genau gewogen (m2). Der Trockengehalt bestimmt sich aus folgenden Gleichung:

$$\text{Trockengehalt in \%} = [(m2 - w1)/ m1] \times 100$$

Die Anzahl der Prüfungen beträgt mindestens n = 3.

Bestimmung der relativen Aufnahmekapazität und der relativen Retentionskapazität der absorbierenden Struktur in Anlehnung an den Teebeuteltest.

An Prüfgeräten und Reagenzien werden benötigt: Teebeutelvlies von Degussa Evonik, Vliesschweißgerät, Analysenwaage mit Ablesegenauigkeit 0,0001 g, Präzisionswaage mit Ablesegenauigkeit 0,01 g, Schale, Abtropfgitter mit Klammern, Stoppuhr, Laborzentrifuge (1111 U/min, Durchmesser 40 cm, entspricht einer Beschleunigung von 276 g mit g= 9,81m/s$^2$) und 0,9% Natriumchloridlösung.

Die Probenvorbereitung und Prüfung erfolgt im Normklima bei 23°C $\pm$ 2 °C / 50% relative Luftfeuchte.

Zur Probenvorbereitung werden Teefilterbeutel aus dem Vlies durch Einfalten des Vlieses um eine Halbachse, Verschweißen entlang der Seitenkanten und Beibehalt einer offenen Seite hergestellt. Probemusterstanzlinge werden von der absorbierenden Struktur entnommen, gewogen (= M1) und in den vorbereiteten Teebeutel eingebracht. Dann wird die offene Seite des Teefilters zugeschweißt. In der gleichen Weise werden leere Teebeutel als Bildprobe verschweißt.

Als Probenmuster wurden Stanzlinge von 145 x 45mm Größe eingesetzt.

Für die Prüfung werden die mit Probenmuster befüllten Teebeutel zum ersten Befeuchten in die NaCl-Lösung gelegt und darin für 30 min untergetaucht. Danach werden die Teebeutel aus der NaCl-Lösung entnommen, um sie dann 10 min mit der Klammer quer an der Schweißnaht frei hängend abtropfen zu lassen, und um sie dann anschließend zu wiegen (= M2).

Anschließend werden die Teebeutel in der Laborzentrifuge 4 min geschleudert und dann erneut gewogen (=M3).

Als Bildproben werden unter gleichen Bedingungen leere Teebeutel geprüft (= M4).

Die Absorption A nach Abtropfen berechnet sich wie folgt:

$$A = (M2 - M4 - M1) / M1 \ [g/g]$$

Die Retention R nach Schleudern berechnet sich wie folgt:

$$R = (M3 - M4 - M1) / M1 \ [g/g]$$

Die Prüfung erfolgt anhand von 5 Einzelmessungen an Proben und 3 Einzelmessungen an Bildproben und die Angabe erfolgt als Mittelwert aus diesen Messungen und dabei gerundet auf eine Dezimale.

Weitere Merkmale, Vorteile und Einzelheiten der Erfindung ergeben sich aus den beigefügten Patentansprüchen und aus der zeichnerischen Darstellung und nachfolgenden Beschreibung bevorzugter Ausführungsformen der Erfindung.

Für die vorstehend offenbarten Merkmale und die nachfolgend in den Patentansprüchen zum Ausdruck gebrachten Merkmale wird unabhängig einer etwaigen Rückbeziehung und in beliebiger Kombination miteinander Patentschutz in Anspruch genommen.

Die Erfindung soll im Folgenden anhand von Figuren näher erläutert werden, dabei zeigen

Figur 1    schematisch einen Querschnitt durch eine erfindungsgemäße absorbierende Struktur,

Figur 2    schematisch einen Querschnitt durch eine weitere Ausführungsform einer erfindungsgemäßen absorbierenden Struktur und

Figur 3    schematisch einen Querschnitt durch einen absorbierenden Artikel mit einer erfindungsgemäßen absorbierenden Struktur

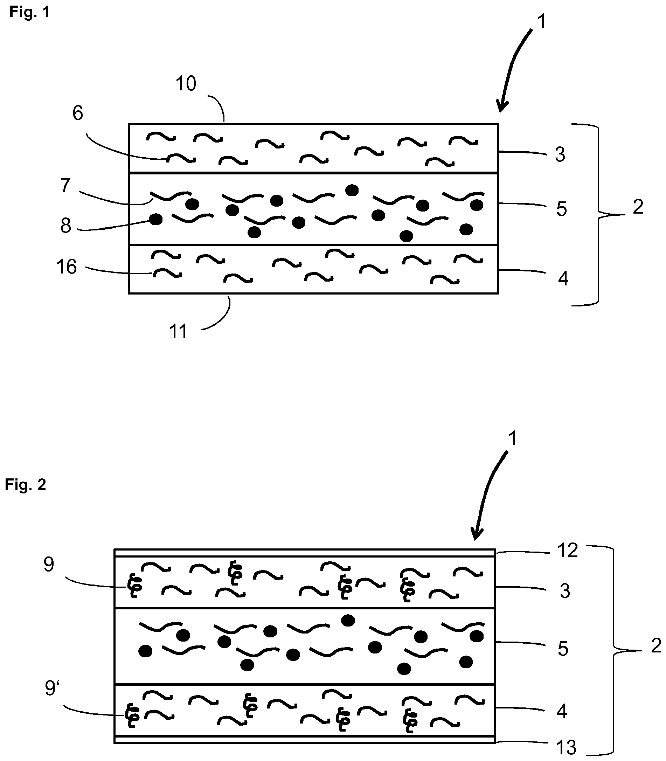

Figur 1 zeigt schematisch im Querschnitt eine bevorzugte absorbierende Struktur 1 mit einer Abfolge 2 an miteinander verbundenen Lagen. Diese Abfolge umfasst eine erste äußere absorbierende Lage 3 aus Airlaid-Material, eine zweite äußere absorbierende Lage 4 aus Airlaid-Material und eine zwischen diesen beiden Lagen angeordnete Flüssigkeitsspeicherlage 5 aus Airlaid-Material. Dabei weist die erste äußere Lage 3 erste Cellulose-Fasern 6, die zweite äußere Lage 4 zweite Cellulose-Fasern 16 und die Flüssigkeitsspeicherlage 5 dritte Cellulose-Fasern 7 und superabsorbierende Komponenten 8 auf. Wesentlich dabei ist, dass sich die ersten und zweiten Cellulose-Fasern 6, 16 in ihrem pH-Wert vom pH-Wert der dritten Cellulose-Fasern 7 unterscheiden. Der pH-Wert der ersten und zweiten Cellulose-Fasern 6, 16 ist geringer als der pH-Wert der zweiten Cellulose-Fasern 7. Der pH-Wert der ersten und zweiten Cellulose-Fasern ist dabei kleiner pH 5,0. Weiter vorzugsweise ist der pH-Wert der ersten und/oder zweiten Cellulose-Fasern kleiner 4,7, vorzugsweise kleiner 4,5, vorzugsweise kleiner 4,2, und weiter vorzugsweise größer 3,2, weiter vorzugsweise größer 3,5, weiter vorzugsweise größer 3,7, weiter vorzugsweise größer 3,9. Der pH-Wert der Cellulose-Fasern wird nach der dargelegten Methode bestimmt. Durch die Ausstattung beider äußeren absorbierenden Lagen mit sauren ersten bzw. zweiten Cellulose-Fasern 6, 16 kann die absorbierende Struktur 1 in der direkten Anwendung sowie innerhalb eines absorbierenden Artikels, wie beispielsweise einer Inkontinenzvorlage, flexibel angeordnet sein. Unabhängig von der Orientierung der absorbierenden Struktur ist stets eine saure Cellulose-Fasern aufweisende äußere absorbierende Lage zur Haut des Anwenders gerichtet und kann die den Lagen zugeschriebenen positiven wachstumshemmenden Eigenschaften gegenüber Mikroorganismen wirken lassen. Der pH-Wert der ersten und/oder zweiten Cellulose-Fasern unterscheidet sich vom pH-Wert der dritten Cellulose-Fasern vorzugsweise um mindestens 0,5 und höchstens 3,0. Vorzugsweise unterscheidet sich der pH-Wert der ersten und/oder zweiten Cellulose-Fasern vom pH-Wert der dritten Cellulose-Fasern um höchstens 2,0.

Bei den ersten, zweiten und dritten Cellulose-Fasern wird als cellulosisches Material vorzugsweise Fluffpulp eingesetzt. Als erste und/oder zweite Cellulose-Fasern können vorzugsweise Reaktionsprodukte aus dem cellulosischen Material Fluffpulp mit Polycarbonsäuren, wie beispielsweise Citronensäure, eingesetzt werden.

Den Airlaid-Materialien der Lagen 3, 4, 5 können durchaus andere Stapelfasern beigemengt sein, jedoch ist erfindungsgemäß in den Airlaid-Materialien keine Fasermischung mit Meltblown Fasern oder Spunbond-Fasern vorgesehen, sondern nur Fasern von endlicher Länge. So können in einer bevorzugten Ausführungsform der absorbierenden Struktur 1, wie schematisch in Figur 2 dargestellt, im Airlaid-Material der ersten und der zweiten äußeren absorbierenden Lage 3, 4 Bindefasern 9, 9', insbesondere in Form von Bikomponentenfasern aus Polyethylentherephthalat und Polyethylen enthalten sein. Die Bindefasern tragen vorteilhaft zur Festigkeit der äußeren absorbierenden Lagen bei. Aber auch für die Differenzierung der Funktionalität der äußeren absorbierenden Lagen gegenüber den dazu mittig angeordneten Lagen, wie eben die Flüssigkeitsspeicherlage, tragen Bindefasern vorteilhaft zu einer Weiterleitung der Flüssigkeit in Richtung Mitte zur Flüssigkeitsspeicherlage 5 bei. An der ersten und zweiten Oberseite 10, 11 der Abfolge 2 an den miteinander verbundenen Lagen kann zusätzlich eine Bindemittelbeschichtung 12, 13 aus polymeren Materialien aufgetragen sein, die vorteilhaft zur Abriebfestigkeit der absorbierenden Struktur als auch zur Transferfunktion der Flüssigkeit ins Innere der absorbierenden Struktur beiträgt. Die Bindemittelbeschichtung kann beispielsweise auf Basis eines Ethylen-Vinylacetat-Copolymers sein.

In einer bevorzugten Ausführungsform der absorbierenden Struktur 1 sind die Lagen in der Abfolge 2 spiegelsymmetrisch. Insbesondere besteht die Abfolge 2 aus einer ersten und zweiten äußeren absorbierenden Lage 3, 4 und der dazwischen angeordneten Flüssigkeitsspeicherlage 5, wie in der Figur 1 oder 2 schematisch dargestellt ist.

In einer besonders bevorzugten Ausführungsform der absorbierenden Struktur 1 besteht die Abfolge 2 an miteinander verbundenen Lagen aus der ersten und zweiten äußeren absorbierenden Lage 3, 4 und der dazwischen angeordneten Flüssigkeitsspeicherlage 5. Die ersten Cellulose-Fasern 6 im Airlaid-Material der ersten äußeren absorbierenden Lage 3 und die zweiten Cellulose-Fasern 16 im Airlaid-Material der zweiten äußeren absorbierenden Lage 4 sind aus Fluffpulp gebildet und weisen einen pH-Wert von 3,9 - 4,2 auf. Die dritten Cellulose-Fasern 7 sind ebenso aus Fluffpulp gebildet, aber mit einem pH-Wert von 5,9 - 6,2. Der Anteil an ersten und zweiten Cellulose-Fasern 6, 16 der ersten und zweiten äußeren absorbierenden Lage 3, 4 in Summe beträgt 30 - 40 Gew-% in Bezug auf das Gesamtgewicht der Abfolge 2 an miteinander verbundenen Strukturen. Die superabsorbierenden Komponenten 8 sind ausschließlich im Airlaid-Material der Flüssigkeitsspeicherlage 5, und in einem Anteil von 40 - 50 Gew-% bezogen auf das Gesamtgewicht der Abfolge 2 eingebracht. Eine Beimengung an Bindefasern 9, 9' in Form von Bikomponentenfasern ist in den beiden äußeren absorbierenden Lagen 3, 4 und dabei in Summe in einem Anteil von 4 - 6 Gew-% bezogen auf das Gesamtgewicht der Abfolge 2 vorgesehen. Auf die beiden Oberseiten 10, 11 der Abfolge 2 ist eine Bindemittelbeschichtung 12, 13 in einem Anteil von höchstens 5 Gew-% aufgebracht. Vorzugsweise weist die absorbierende Struktur 1 ein Flächengewicht von mindestens 320 g/m$^2$ und von höchstens 400 g/m$^2$ bei einer Dicke von 2- 4 mm auf. Die relative Absorptionskapazität der absorbierenden Struktur 1 bemisst sich vorzugsweise auf 22 - 24 g/g und die relative Retentionskapazität auf vorzugsweise 9 - 12 g/g.

Bei einer besonders bevorzugten Ausführung sind die beiden äußeren absorbierenden Lagen zumindest hinsichtlich des pH-Werts der ersten und zweiten Cellulose-Fasern identisch, weiter bevorzugt sind die ersten und zweiten Cellulose-Fasern in deren chemischen Zusammensetzung oder deren Ursprung oder Herkunft identisch, insbesondere bevorzugt sind die beiden äußeren absorbierenden Lagen 3, 4 hinsichtlich aller Eigenschaften identisch.

Mit dieser Ausführungsform der absorbierenden Struktur kann die absorbierende Struktur innerhalb eines absorbierenden Artikels, wie in einer Inkontinenzvorlage, entweder mit der ersten absorbierenden Lage 3 körperzugewandt oder auch mit der zweiten absorbierenden Lage 4 körperzugewandt angeordnet sein und somit flexibel, auch in einem laufenden Herstellungsverfahren eingesetzt werden.

Die erfindungsmäße absorbierende Struktur 1 kann als solche bereits als ein absorbierender Artikel eingesetzt werden. Die erfindungsmäße absorbierende Struktur kann vorzugsweise für die Konfektionierung eines bestimmten absorbierenden Artikels, wie insbesondere einer Damenhygienebinde, einer Inkontinenzvorlage, einer Inkontinenzwindel offenen oder geschlossenen Typs oder einer Wundauflage eingesetzt werden.

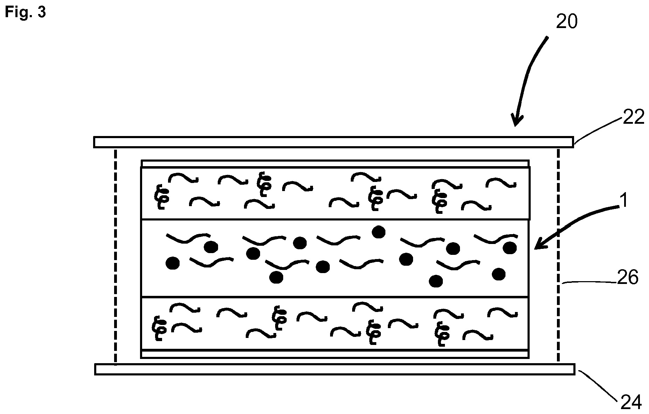

Für einen absorbierenden Hygieneartikel, wie eine Damenhygienebinde, eine Inkontinenzvorlage, eine Inkontinenzwindel offenen oder geschlossenen Typs können an den äußeren absorbierenden Lagen 3, 4 weitere Lagen ergänzend angeordnet werden, wie schematisch in der Figur 3 gezeigt ist. So ist an der Oberseite der einen äußeren absorbierenden Lage als der späteren körperzugewandten Lage eine flüssigkeitsdurchlässige Lage 22, insbesondere auf Basis eines Vliesstoffes und an der Oberseite der anderen äußeren absorbierenden Lage als der späteren körperabgewandten Lage eine flüssigkeitsundurchlässige Lage 24, insbesondere auf Basis von Folien, angeordnet. Die flüssigkeitsdurchlässige und die flüssigkeitsundurchlässige Lage 22, 24 erstrecken sind vorzugsweise über die Kanten der absorbierenden Struktur 1 hinaus und sind mittels Fixiermittel 26, wie beispielsweise Kleber oder Schweißnaht, miteinander verbunden.

Prüfung auf antimikrobielle Aktivität der ersten oder zweiten Cellulose-Fasern, angelehnt an die Flüssigmethode nach AATCC 174- 2007

Das Prüfverfahren dient zur quantitativen Bestimmung der antimikrobiellen Wirkung der ersten oder zweiten Cellulose-Fasern. Dazu wird das in Ringerlösung aufgenommene Cellulose-Fasermaterial mit dem jeweiligen Bakterienstamm beimpft. Nach definierten Zeiten werden Keimzahlbestimmungen durchgeführt.

Für den Versuch werden benötigt:

- Sterile Schere, sterile Pinzette
- Sterile Flaschen, Petrischalen, Reagenzgläser
- Brutschrank (37°C $\pm$ 2 °C)
- Ringerlösung: Die Ringerlösung wird durch Auflösen von 1 Ringer-Tablette (erhältlich bei der Fa. Merck unter der Materialnummer 1.15525.0001) in 500 ml destilliertem Wasser hergestellt. Eine Ringertablette enthält 0,00525 g Ammoniumchlorid, 0,005 g Natriumhydrogencarbonat, 0,04 g Calciumchlorid - 2 Hydrat, 0,00525 g Kaliumchlorid und 1,125 g Natriumchlorid.
- Ringerlösung mit Enthemmern (0,3% Lecithin, 0,1% Histidin, 1% Tween).
- CASO-Agar = Caseinpepton-Sojamehlpepton-Agar (erhältlich bei der Fa. Merck unter der Materialnummer 1.05458.0500)
- CASO-Bouillon (erhältlich bei der Fa. Merck unter der Materialnummer 1.05459.0500)
- Bakterienstämme: Staphylococcus aureus ATCC 6538 (DSM 346), Klebsiella pneumoniae ATCC 4352 (DSM 789)

Vorbereitung:

Die Bakterienkultur für das Beimpfen der Proben wird aus einer der Stammkultur entnommenen Impfkultur und deren Anreicherung in einer CASO-Bouillon für 18 - 24 Stunden gewonnen. Für den Versuch wird der jeweilige Bakterienstamm mit Kolonien von 1-5 x $10^6$ KBE/ml (KBE = Kolonie bildende Einheiten) eingesetzt.

Jede Probe wird vor der Versuchsdurchführung und vor der Beimpfung mit den Keimen mittels Ethylenoxid in einem validierten Verfahren (780 mg/l; 240min, 43-45 °C) sterilisiert.

Unter Proben werden dabei die Cellulose-Fasern enthaltenen Ansätze sowie die KontrollAnsätze ohne Cellulose-Fasern verstanden.

Von den Cellulose-Fasern wird jeweils ein Probenansatz mit definiertem Fasermaterial in definiertem Volumen an Ringer-Lösung angesetzt. Im Prüfverfahren wird die eingesetzte Keimzahl zusätzlich überprüft, d.h. sämtliche Prüfverfahrensschritte werden ohne Einbringen von Cellulose-Fasern durchgeführt (die sogenannte Keimzahl-Kontrolle). Des Weiteren wird ein Puffer von pH 3,7 (mit HCl-Lösung auf pH 3,7 eingestellte 0,9% NaCl - Lösung) ohne Einsatz von Cellulose-Fasern mitgeführt.

Pro Probe ist eine Dreifachbestimmung zu den Prüfzeitpunkten Zeit 1= 0 h (also sofort, direkt nach Beimpfen des Probenansatzes mit Bakterienstämme), Zeit 2= 4 h und Zeit 3 = 24 h vorgesehen.

Versuchsdurchführung:

Zur Keimzahlbestimmung wird jeder Probenansatz in eine sterile 250 ml Schraubverschlussflasche gegeben und mit 1 ml der Bakterienkultur (1-5 x $10^6$ KBE/ml) beimpft. Dann wird 100 ml an steriler Ringerlösung zugegeben.

Für die Bestimmung des Sofort-Werts, also Zeit 1 = 0 h, wird gleich nach Zugabe der Ringerlösung 1 ml des Probenansatzes entnommen und auf einer sterilen Petrischale mit CASO-Agar ausplattiert. Die Probenansätze als solche werden dann in den Flaschen im Brutschrank bei 37°C $\pm$ 2 °C bebrütet und für die weiteren definierten Zeitpunkten entsprechend weiter verarbeitet:

Nach den jeweiligen Wachstumszeitpunkten (0h, 4h, 24h) werden die Schraubverschlussgefäße für 1 min im Ultraschallbad behandelt und anschließend für 1 min mechanisch geschüttelt. Nachfolgend wird daraus eine Verdünnungsreihe angelegt, indem jeweils 1 ml aus der vorangehenden Lösung in ein steriles Röhrchen mit vorgelegten 9 ml an Ringerlösung mit Enthemmer eingebracht wird. Die Verdünnungsreihen werden derart angelegt, so dass eine spätere Auszählung der herangewachsenen Kolonien möglich ist. Bei Ansätzen mit zu erwartendem höheren Bakterienwachstum, wie den Keimzahl-Kontrollen, sind demnach höhere Verdünnungen anzusetzen.

Anschließend wird mit einer sterilen Pipette aus jeder Verdünnungsstufe 1 ml entnommen und in eine sterile Petrischale pipettiert und mit 15 - 20 ml Agarmedium (CASO-Agar) beschichtet. Zur gleichmäßigen Verteilung des Mediums werden die befüllten Petrischalen kreisend über die Arbeitsfläche bewegt. Nach Erstarren des Agars werden die Petrischalen mit dem Deckel nach unten in den Brutschrank gestellt und 18 - 24 Stunden bei 37 ± 2°C im Brutschrank bebrütet.

Die Auswertung der Keimzahlen erfolgt dann mittels Abzählen der gewachsenen Keimkolonien. Bei stärkerem Kolonienwachstum sind die Platten mit Kolonien zwischen 30 und 300 zur Auswertung heranzuziehen und möglichst die Zählwerte zweier Verdünnungsstufen zu berücksichtigen.

Die mittlere geschätzte Kolonienzahl ermittelt sich wie folgt:

$$x_m = \frac{Summe \quad Koloniezählwert}{Summe \quad Verdünnungsstufen}$$

$$\text{bspw.} \quad x_m = \frac{303 + 290 + 32 + 28}{10^{-4} + 10^{-4} + 10^{-5} + 10^{-5}} = \frac{653}{2{,}2 \times 10^{-4}} = 2{,}97 \times 10^6$$

Die ermittelten Werte werden dann in der Einheit KBE/ml angegeben.

Beispiel: Prüfung der antimikrobiellen Aktivität von ersten oder zweiten Cellulose-Fasern im Vergleich zu dritten Cellulose-Fasern

Die Cellulose-Fasern und die Reagenzien werden vor Beimpfen wie oben beschrieben sterilisiert.

Es werden als Probenansätze gewählt:

- 4g erste oder zweite Cellulose-Fasern mit einem pH-Wert von 3,9 - 4,1 ad 100 ml Ringerlösung ohne Enthemmer
- 4g dritte Cellulose-Fasern mit einem pH-Wert von 5,9 - 6,2 ad 100 ml Ringerlösung ohne Enthemmer
- Keimzahl-Kontrolle: 100 ml Ringerlösung ohne Enthemmer
- pH-Wert-Kontrolle: 100 ml Puffer pH 3,7 (mit HCl-Lösung auf pH 3,7 eingestellte 0,9% NaCl - Lösung)

Die Versuchsdurchführung erfolgt dann wie oben beschrieben. Jeder Probenansatz wird mit 1 ml der Bakterienkultur (1-5 x $10^6$ KBE/ml) beimpft. Die Keimzahlbestimmungen erfolgen nach den Inkubationszeiten Zeit 1 = 0 h, Zeit 2 = 4 h und Zeit 3 = 24 h.

Die Ergebnisse in Tabelle 1 bzw. Diagramm 1 und Tabelle 2 bzw. Diagramm 2 zeigen die antimikrobielle Wirkung der ersten oder zweiten Cellulose-Fasern im Vergleich zum Verhalten der dritten Cellulose-Fasern.

Tabelle 1: Antimikrobielle Wirksamkeit bei Versuchskeim Staphylococcus aureus DSM 346

|  |  | Nach 0 h (sofort) [KBE/ml] | Nach 4 h [KBE/ml] | Nach 24 h [KBE/ml] |
|---|---|---|---|---|
| Keimzahl-Kontrolle | P1 | 1,20E+07 | 2,10E+07 | 5,30E+07 |
| Dritte Cellulose-Fasern | P2 | 1,60E+07 | 9,50E+06 | 1,00E+07 |
| Erste oder Zweite Cellulose-Fasern | P3 | 6,10E+06 | 1,50E+06 | 6,70E+02 |
| Puffer pH 3,7 | P4 | 9,40E+06 | 2,60E+06 | 1,00E+02 |

Tabelle 2: Antimikrobielle Wirksamkeit bei Versuchskeim Klebsiella pneumoniae DSM 789

|  |  | Nach 0 h (sofort) [KBE/ml] | Nach 4 h [KBE/ml] | Nach 24 h [KBE/ml] |
|---|---|---|---|---|
| Keimzahl-Kontrolle | P1 | 1,10E+07 | 1,70E+07 | 1,30E+08 |
| Dritte Cellulose-Fasern | P2 | 1,50E+07 | 1.00E+08 | 9,90E+08 |
| Erste oder Zweite Cellulose-Fasern | P3 | 1,60E+07 | 1,30E+07 | 8,90E+05 |
| Puffer pH 3,7 | P4 | 1,50E+07 | 1,30E+07 | 2,80E+06 |

Diagramm 1:

Staphylococcus aureus DSM 346

Diagramm 2:

Klebsiella pneumoniae DSM 789

**Patentansprüche**

1. Absorbierende Struktur (1) mit einer Abfolge (2) an miteinander verbundenen Lagen umfassend eine erste äußere absorbierende Lage (3) aus Airlaid-Material, eine zweite äußere absorbierende Lage (4) aus Airlaid-Material und eine zwischen der ersten und zweiten äußeren absorbierenden Lage angeordnete Flüssigkeitsspeicherlage (5) aus Airlaid-Material, **dadurch gekennzeichnet, dass** das Airlaid-Material der ersten äußeren absorbierenden Lage (3) erste Cellulose-Fasern (6), das Airlaid-Material der zweiten äußeren absorbierenden Lage (4) zweite Cellulose-Fasern (16) und das Airlaid-Material der Flüssigkeitsspeicherlage (5) dritte Cellulose-Fasern (7) und superabsorbierende Komponenten (8) aufweisen, wobei die ersten und die zweiten Cellulose-Fasern (6, 16) einen geringeren pH-Wert aufweisen als die dritten Cellulose-Fasern (7) und der pH-Wert der ersten und der zweiten Cellulose-Fasern (6, 16) kleiner 5,0 ist.

2. Absorbierende Struktur (1) nach Anspruch 1, **dadurch gekennzeichnet, dass** die ersten und/oder die zweiten Cellulose-Fasern (6, 16) einen pH-Wert kleiner 4,7, vorzugsweise kleiner 4,5, vorzugsweise kleiner 4,2, und weiter vorzugsweise einen pH-Wert größer 3,2, weiter vorzugsweise größer 3,5, weiter vorzugsweise größer 3,7, weiter vorzugsweise größer 3,9 aufweisen.

3. Absorbierende Struktur (1) nach Anspruch 1 oder 2, **dadurch gekennzeichnet, dass** die dritten Cellulose-Fasern (7) einen pH-Wert kleiner pH 7,0, bevorzugt kleiner 6,7, weiter bevorzugt kleiner 6,5, weiter bevorzugt kleiner 6,3, und weiter bevorzugt größer pH 5,0, weiter bevorzugt größer 5,5, weiter bevorzugt größer 5,7, weiter bevorzugt größer 5,9 aufweisen.

4. Absorbierende Struktur (1) nach einem der vorangehenden Ansprüche, **dadurch gekennzeichnet, dass** der pH-Wert der ersten und/oder der zweiten Cellulose-Fasern (6) sich von dem pH-Wert der dritten Cellulose-Fasern (7) um mindestens 0,5, bevorzugt mindestens 0,7, weiter bevorzugt mindestens 0,9, weiter bevorzugt mindestens 1,0, weiter bevorzugt höchstens 3,0, weiter bevorzugt höchstens 2,7, weiter bevorzugt höchstens 2,5, weiter bevorzugt höchstens 2,0 unterscheidet.

5. Absorbierende Struktur (1) nach einem der vorangehenden Ansprüche, **dadurch gekennzeichnet, dass** das Airlaid-Material der Flüssigkeitsspeicherlage (5) keine ersten und keine zweiten Cellulose-Fasern (6) aufweist.

6. Absorbierende Struktur (1) nach einem der vorangehenden Ansprüche, **dadurch gekennzeichnet, dass** das Airlaid-Material der ersten und/oder zweiten äußeren absorbierenden Lage (3, 4) keine dritten Cellulose-Fasern (7) aufweist.

7. Absorbierende Struktur (1) nach einem der vorangehenden Ansprüche, **dadurch gekennzeichnet, dass** die ersten und die zweiten Cellulose-Fasern (6, 16) der ersten und zweiten äußeren absorbierenden Lage in Summe in einem Anteil von 20 - 50 Gew-%, bevorzugt von 25 - 45 Gew-%, weiter bevorzugt von 30- 40 Gew-% bezogen auf das Gesamtgewicht der Abfolge (2) an miteinander verbundenen Lagen enthalten sind.

8. Absorbierende Struktur (1) nach einem der vorangehenden Ansprüche, **dadurch gekennzeichnet, dass** die ersten und/oder die zweiten Cellulose-Fasern (6, 16) und/oder die dritten Cellulose-Fasern (7) aus cellulosischem Fasermaterial aus der Gruppe Baumwollfasern und/oder Fluffpulp bestehen oder solches enthalten.

9. Absorbierende Struktur (1) nach einem der vorangehenden Ansprüche, **dadurch gekennzeichnet, dass** die ersten und/oder die zweiten Cellulose-Fasern (6, 16) Reaktionsprodukte aus cellulosischem Fasermaterial mit Polycarbonsäuren oder Salzen davon und/oder Polyacrylsäuren oder Salzen davon sind.

10. Absorbierende Struktur (1) nach einem der vorangehenden Ansprüche, **dadurch gekennzeichnet, dass** das Airlaid-Material der ersten und/oder zweiten äußeren absorbierenden Lage (3, 4) keine superabsorbierenden Komponenten (8) aufweist.

11. Absorbierende Struktur (1) nach einem der vorangehenden Ansprüche, **dadurch gekennzeichnet, dass** die superabsorbierenden Komponenten (8) der Flüssigkeitsspeicherschicht (5) in einem Anteil von mindestens 25 Gew-%, bevorzugt von mindestens 30 Gew-%, bevorzugt von mindestens 35 Gew-%, weiter bevorzugt von mindestens 40 Gew-%, weiter bevorzugt von höchstens 70 Gew-%, weiter bevorzugt von höchstens 60 Gew-%, weiter bevorzugt von höchstens 50 Gew-% bezogen auf das Gesamtgewicht der Abfolge (2) an miteinander verbundenen Lagen enthalten sind.

12. Absorbierende Struktur (1) nach einem der vorangehenden Ansprüche, **dadurch gekennzeichnet, dass** das Airlaid-Material der ersten und/oder zweiten äußeren absorbierenden Lage (3, 4) Bindemittel, bevorzugt Bindefasern (9, 9'), weiter bevorzugt Bindefasern in Form von Bi- und/oder Mehrkomponentenfasern, aufweist.

13. Absorbierende Struktur (1) nach einem der vorangehenden Ansprüche, **dadurch gekennzeichnet, dass** das Airlaid-Material der Flüssigkeitsspeicherlage (5) keine Bindefasern (9, 9'), bevorzugt keine Bindemittel aufweist.

14. Absorbierende Struktur (1) nach einem der vorangehenden Ansprüche, **dadurch gekennzeichnet, dass** im Airlaid-Material der ersten äußeren und/oder der zweiten äußeren absorbierenden Lage (3, 4) Bindefasern (9, 9') in Summe mit einem Anteil von 2 - 10 Gew-%, bevorzugt von 2 - 7 Gew-%, weiter bevorzugt von 2 - 6 Gew-%, weiter bevorzugt 3 - 6 Gew-%, weiter bevorzugt 4 - 6 Gew-% bezogen auf das Gesamtgewicht der Abfolge (2) an miteinander verbundenen Lagen enthalten sind.

15. Absorbierende Struktur (1) nach einem der vorangehenden Ansprüche, **dadurch gekennzeichnet, dass** die Abfolge (2) an miteinander verbundenen Lagen eine erste und zweite äußere Oberseite (10, 11) aufweist und die erste und/oder zweite äußere Oberseite (10,11) eine Bindemittelbeschichtung (12,13) aufweist.

**16.** Absorbierende Struktur (1) nach einem der vorangehenden Ansprüche, **dadurch gekennzeichnet, dass** die erste und zweite äußere absorbierende Lage (3, 4) zumindest hinsichtlich einer Eigenschaft identisch sind, dabei mit der Eigenschaft entnommen aus der Gruppe enthaltend Flächengewicht, pH-Wert der ersten und der zweiten Cellulose-Fasern, chemische Zusammensetzung oder natürliche Herkunft oder Ursprung der ersten und zweiten Cellulose-Fasern, Gewichtsanteil an ersten und zweiten Cellulose-Fasern, Gewichtsanteil an Bindefasern, eingesetzte Fasermischung des Airlaid-Materials.

**17.** Absorbierende Struktur (1) nach Anspruch 16, **dadurch gekennzeichnet, dass** die erste und die zweite äußere absorbierende Lage (3, 4) zumindest hinsichtlich des pH-Werts der ersten Cellulose-Fasern (6) und des pH-Werts der zweiten Cellulose-Fasern (16) identisch sind.

**18.** Absorbierende Struktur (1) nach Anspruch 16, **dadurch gekennzeichnet, dass** die erste und zweite äußere absorbierende Lage (3, 4) hinsichtlich pH-Wert der ersten und der zweiten Cellulose-Fasern (6, 16) und hinsichtlich des Gewichtsanteils an ersten und an zweiten Cellulose-Fasern (6, 16) identisch sind.

**19.** Absorbierende Struktur (1) nach Anspruch 16, **dadurch gekennzeichnet, dass** die erste und zweite äußere absorbierende Lage (3, 4) hinsichtlich pH-Wert der ersten und der zweiten Cellulose-Fasern (6, 16) und hinsichtlich der chemischen Zusammensetzung oder der natürlichen Herkunft oder des Ursprungs der ersten und zweiten Cellulose-Fasern identisch sind.

**20.** Absorbierende Struktur (1) nach Anspruch 16, **dadurch gekennzeichnet, dass** die erste und zweite äußere absorbierende Lage (3, 4) hinsichtlich pH-Wert der ersten und der zweiten Cellulose-Fasern (6, 16), hinsichtlich der chemischen Zusammensetzung oder der natürlichen Herkunft oder des Ursprungs der ersten und zweiten Cellulose-Fasern und hinsichtlich des Gewichtsanteils an ersten und an zweiten Cellulose-Fasern (6, 16) identisch sind.

**21.** Absorbierende Struktur (1) nach Anspruch 16, **dadurch gekennzeichnet, dass** die erste und zweite äußere absorbierende Lage (3, 4) hinsichtlich aller Eigenschaften identisch sind.

**22.** Absorbierende Struktur (1) nach einem der vorangehenden Ansprüche, **dadurch gekennzeichnet, dass** die Abfolge (2) an miteinander verbundenen Lagen spiegelsymmetrisch ist.

**23.** Absorbierende Struktur (1) nach einem der vorangehenden Ansprüche, **dadurch gekennzeichnet, dass** die absorbierende Struktur (1) einen absorbierenden Artikel (20) bildet oder in einem absorbierenden Artikel (20) enthalten ist.

**24.** Absorbierender Artikel (20) umfassend eine absorbierende Struktur (1) nach einem der vorstehenden Ansprüche 1 bis 22.

**25.** Absorbierende Artikel (20) nach 24, **dadurch gekennzeichnet, dass** der absorbierende Artikel eine Inkontinenzvorlage, eine Damenhygienebinde, eine Inkontinenzwindel offenen Typs mit Verschlusssystemen, eine Inkontinenzwindel geschlossenen Typs, und/oder eine Wundauflage ist.

## Claims

**1.** Absorbent structure (1) having a sequence (2) of interconnected plies comprising a first outer absorbent ply (3) of airlaid material, a second outer absorbent ply (4) of airlaid material and disposed between the first and second outer absorbent plies a liquid storage ply (5) of airlaid material, **characterized in that** the airlaid material of the first outer absorbent ply (3) includes first cellulose fibres (6), the airlaid material of the second outer absorbent ply (4) includes second cellulose fibres (16) and the airlaid material of the liquid storage ply (5) includes third cellulose fibres (7) and superabsorbent components (8), wherein the first and second cellulose fibres (6, 16) have a lower pH than the third cellulose fibres (7) and the pH of the first and second cellulose fibres (6, 16) is below 5.0.

**2.** Absorbent structure (1) according to Claim 1, **characterized in that** the first and/or second cellulose fibres (6, 16) have a pH below 4.7, preferably below 4.5, preferably below 4.2 and more further preferably a pH above 3.2, further preferably above 3.5, further preferably above 3.7, further preferably above 3.9.

3. Absorbent structure (1) according to Claim 1 or 2, **characterized in that** the third cellulose fibres (7) have a pH below 7.0, preferably below 6.7, further preferably below 6.5, further preferably below 6.3, and further preferably above pH 5.0, further preferably above 5.5, further preferably above 5.7, further preferably above 5.9.

4. Absorbent structure (1) according to any preceding claim, **characterized in that** the pH of the first and/or second cellulose fibres (6) differs from the pH of the third cellulose fibres (7) by not less than 0.5, preferably not less than 0.7, further preferably not less than 0.9, further preferably not less than 1.0, further preferably not more than 3.0, further preferably not more than 2.7, further preferably not more than 2.5, further preferably not more than 2.0.

5. Absorbent structure (1) according to any preceding claim, **characterized in that** the airlaid material of the liquid storage ply (5) includes no first and no second cellulose fibres (6).

6. Absorbent structure (1) according to any preceding claim, **characterized in that** the airlaid material of the first and/or second outer absorbent plies (3, 4) includes no third cellulose fibres (7).

7. Absorbent structure (1) according to any preceding claim, **characterized in that** the first and second cellulose fibres (6, 16) of the first and second outer absorbent plies together comprise 20-50 wt%, preferably 25-45 wt%, further preferably 30-40 wt% based on the combined weight of the sequence (2) of interconnected plies.

8. Absorbent structure (1) according to any preceding claim, **characterized in that** the first and/or second cellulose fibres (6, 16) and/or the third cellulose fibres (7) consist of or contain cellulosic fibrous material from the group of cotton fibres and/or fluff pulp.

9. Absorbent structure (1) according to any preceding claim, **characterized in that** the first and/or second cellulose fibres (6, 16) are reaction products of cellulosic fibrous material with polycarboxylic acids or salts thereof and/or polyacrylic acids or salts thereof.

10. Absorbent structure (1) according to any preceding claim, **characterized in that** the airlaid material of the first and/or second outer absorbent plies (3, 4) includes no superabsorbent components (8).

11. Absorbent structure (1) according to any preceding claim, **characterized in that** the superabsorbent components (8) of the liquid storage layer (5) comprise not less than 25 wt%, preferably not less than 30 wt%, preferably not less than 35 wt%, further preferably not less than 40 wt%, further preferably not more than 70 wt%, further preferably not more than 60 wt%, further preferably not more than 50 wt% based on the combined weight of the sequence (2) of interconnected plies.

12. Absorbent structure (1) according to any preceding claim, **characterized in that** the airlaid material of the first and/or second outer absorbent plies (3, 4) includes binding agents, preferably binding fibres (9, 9'), further preferably binding fibres in the form of bi- and/or more-component fibres.

13. Absorbent structure (1) according to any preceding claim, **characterized in that** the airlaid material of the liquid storage ply (5) includes no binding fibres (9, 9'), preferably no binding agents.

14. Absorbent structure (1) according to any preceding claim, **characterized in that** the airlaid material of the first and/or second outer absorbent plies (3, 4) includes binding fibres (9, 9') in a total proportion of 2-10 wt%, preferably 2-7 wt%, further preferably 2-6 wt%, further preferably 3-6 wt%, further preferably 4-6 wt% based on the combined weight of the sequence (2) of interconnected plies.

15. Absorbent structure (1) according to any preceding claim, **characterized in that** the sequence (2) of interconnected plies includes first and second outer surfaces (10, 11) and the first and/or second outer surfaces (10, 11) include a binding agent coating (12, 13).

16. Absorbent structure (1) according to any preceding claim, **characterized in that** the first and second outer absorbent plies (3, 4) are identical with regard to one property at least, this property being taken from the group containing basis weight, pH of first and second cellulose fibres, chemical composition or natural provenience or origin of first and second cellulose fibres, weight fraction of first and second cellulose fibres, weight fraction of binding fibres, fibre blend used for airlaid material.

**17.** Absorbent structure (1) according to Claim 16, **characterized in that** the first and second outer absorbent plies (3, 4) are at least identical with regard to the pH of the first cellulose fibres (6) and the pH of the second cellulose fibres (16).

**18.** Absorbent structure (1) according to Claim 16, **characterized in that** the first and second outer absorbent plies (3, 4) are identical with regard to the pH of the first and second cellulose fibres (6, 16) and with regard to the weight fraction of first and second cellulose fibres (6, 16).

**19.** Absorbent structure (1) according to Claim 16, **characterized in that** the first and second outer absorbent plies (3, 4) are identical with regard to the pH of the first and second cellulose fibres (6, 16) and with regard to the chemical composition or the natural provenience or the origin of the first and second cellulose fibres.

**20.** Absorbent structure (1) according to Claim 16, **characterized in that** the first and second outer absorbent plies (3, 4) are identical with regard to the pH of the first and second cellulose fibres (6, 16) and with regard to the chemical composition or the natural provenience or the origin of the first and second cellulose fibres and with regard to the weight fraction of the first and second cellulose fibres (6, 16).

**21.** Absorbent structure (1) according to Claim 16, **characterized in that** the first and second outer absorbent plies (3, 4) are identical with regard to all properties.

**22.** Absorbent structure (1) according to any preceding claim, **characterized in that** the sequence (2) of interconnected plies has mirror symmetry.

**23.** Absorbent structure (1) according to any preceding claim, **characterized in that** the absorbent structure (1) forms an absorbent article (20) or is included in an absorbent article (20).

**24.** Absorbent article (20) comprising an absorbent structure (1) according to any of preceding Claims 1 to 22.

**25.** Absorbent article (20) according to 24, **characterized in that** the absorbent article is an incontinence pad, a femcare napkin, an incontinence diaper of the open type with closure systems, an incontinence diaper of the closed type, and/or a wound contact material.

**Revendications**

**1.** Structure absorbante (1) présentant une succession (2) de couches reliées les unes aux autres comprenant une première couche (3) absorbante externe en matériau airlaid, une deuxième couche (4) absorbante externe en matériau airlaid et une couche (5) d'accumulation de liquide en matériau airlaid disposée entre la première et la deuxième couche absorbante externe, **caractérisée en ce que** le matériau airlaid de la première couche (3) absorbante externe présente des premières fibres de cellulose (6), le matériau airlaid de la deuxième couche (4) absorbante externe présente des deuxièmes fibres de cellulose (16) et le matériau airlaid de la couche (5) d'accumulation de liquide présente des troisièmes fibres de cellulose (7) et des composants superabsorbants (8), les premières et les deuxièmes fibres de cellulose (6, 16) présentant une valeur de pH inférieure à celle des troisièmes fibres de cellulose (7) et la valeur de pH des premières et des deuxièmes fibres de cellulose (6, 16) étant inférieure à 5,0.

**2.** Structure absorbante (1) selon la revendication 1, **caractérisée en ce que** les premières et/ou les deuxièmes fibres de cellulose (6, 16) présentent une valeur de pH inférieure à 4,7, de préférence inférieure à 4,5, de préférence inférieure à 4,2 et en outre de préférence une valeur de pH supérieure à 3,2, en outre de préférence supérieure à 3,5, en outre de préférence supérieure à 3,7, en outre de préférence supérieure à 3, 9.

**3.** Structure absorbante (1) selon la revendication 1 ou 2, **caractérisée en ce que** les troisièmes fibres de cellulose (7) présentent une valeur de pH inférieure à 7,0, de préférence inférieure à 6,7, en outre de préférence inférieure à 6,5, en outre de préférence inférieure à 6,3 et en outre de préférence supérieure à 5,0, en outre de préférence supérieure à 5,5, en outre de préférence supérieure à 5,7, en outre de préférence supérieure à 5, 9.

**4.** Structure absorbante (1) selon l'une quelconque des revendications précédentes, **caractérisée en ce que** la valeur de pH des premières et/ou des deuxièmes fibres de cellulose (6) se distingue de la valeur de pH des troisièmes fibres de cellulose (7) d'au moins 0,5, de préférence d'au moins 0,7, en outre de préférence d'au moins 0,9, en outre de préférence d'au moins 1,0, en outre de préférence d'au plus 3,0, en outre de préférence d'au plus 2,7, en outre de préférence d'au plus 2,5, en outre de préférence d'au plus 2,0.

**5.** Structure absorbante (1) selon l'une quelconque des revendications précédentes, **caractérisée en ce que** le matériau airlaid de la couche (5) d'accumulation de liquide ne présente pas de premières ni de deuxièmes fibres de cellulose (6).

**6.** Structure absorbante (1) selon l'une quelconque des revendications précédentes, **caractérisée en ce que** le matériau airlaid de la première et/ou de la deuxième couche (3, 4) absorbante externe ne présente pas de troisièmes fibres de cellulose (7).

**7.** Structure absorbante (1) selon l'une quelconque des revendications précédentes, **caractérisée en ce que** les premières et les deuxièmes fibres de cellulose (6, 16) de la première et de la deuxième couche absorbante externe sont contenues, au total, en une proportion de 20-50% en poids, de préférence de 25-45% en poids, en outre de préférence de 30-40% en poids, par rapport au poids total de la succession (2) de couches reliées les unes aux autres.

**8.** Structure absorbante (1) selon l'une quelconque des revendications précédentes, **caractérisée en ce que** les premières et/ou les deuxièmes fibres de cellulose (6, 16) et/ou les troisièmes fibres de cellulose (7) sont constituées par un matériau fibreux cellulosique du groupe formé par les fibres de coton et/ou la pâte défibrillée ou contiennent celui-ci.

**9.** Structure absorbante (1) selon l'une quelconque des revendications précédentes, **caractérisée en ce que** les premières et/ou les deuxièmes fibres de cellulose (6, 16) sont des produits de réaction de matériau fibreux cellulosique avec des poly(acides carboxyliques) ou des sels de ceux-ci et/ou des poly(acides acryliques) ou des sels de ceux-ci.

**10.** Structure absorbante (1) selon l'une quelconque des revendications précédentes, **caractérisée en ce que** le matériau airlaid de la première et/ou de la deuxième couche (3, 4) absorbante externe ne contient pas de composants superabsorbants (8).

**11.** Structure absorbante (1) selon l'une quelconque des revendications précédentes, **caractérisée en ce que** les composants superabsorbants (8) de la couche (5) d'accumulation de liquide sont contenus en une proportion d'au moins 25% en poids, de préférence d'au moins 30% en poids, de préférence d'au moins 35% en poids, en outre de préférence d'au moins 40% en poids, en outre de préférence d'au plus 70% en poids, en outre de préférence d'au plus 60% en poids, en outre de préférence d'au plus 50% en poids, par rapport au poids total de la succession (2) de couches reliées les unes aux autres.

**12.** Structure absorbante (1) selon l'une quelconque des revendications précédentes, **caractérisée en ce que** le matériau airlaid de la première et/ou de la deuxième couche (3, 4) absorbante externe présente des liants, de préférence des fibres de liaison (9, 9'), en outre de préférence des fibres de liaison sous forme de fibres à deux composants et/ou à plus de deux composants.

**13.** Structure absorbante (1) selon l'une quelconque des revendications précédentes, **caractérisée en ce que** le matériau airlaid de la couche (5) d'accumulation de liquide ne présente pas de fibres de liaison (9, 9'), de préférence pas de liant.

**14.** Structure absorbante (1) selon l'une quelconque des revendications précédentes, **caractérisée en ce que** le matériau airlaid de la première et/ou de la deuxième couche (3, 4) absorbante externe contient des fibres de liaison (9, 9'), au total, en une proportion de 2-10% en poids, de préférence de 2-7% en poids, en outre de préférence de 2-6% en poids, en outre de préférence de 3-6% en poids, en outre de préférence de 4-6% en poids, par rapport au poids total de la succession (2) de couches reliées les unes aux autres.

**15.** Structure absorbante (1) selon l'une quelconque des revendications précédentes, **caractérisée en ce que** la succession (2) de couches reliées les unes aux autres présente une première et une deuxième face (10, 11) supérieure externe et la première et/ou la deuxième face (10,11) supérieure externe présente(nt) un revêtement de liant (12,13).

**16.** Structure absorbante (1) selon l'une quelconque des revendications précédentes, **caractérisée en ce que** la première et la deuxième couche (3, 4) absorbante externe sont identiques au moins en ce qui concerne une propriété, la propriété étant choisie dans le groupe contenant le poids surfacique, la valeur de pH des premières et des deuxièmes fibres de cellulose, la composition chimique ou la provenance naturelle ou l'origine des premières et des deuxièmes fibres de cellulose, la proportion pondérale de premières et de deuxièmes fibres de cellulose, la proportion pondérale de fibres de liaison, le mélange de fibres utilisé du matériau airlaid.

**17.** Structure absorbante (1) selon la revendication 16, **caractérisée en ce que** la première et la deuxième couche (3, 4) absorbante externe sont identiques au moins en ce qui concerne la valeur de pH des premières fibres de cellulose (6) et la valeur de pH des deuxièmes fibres de cellulose (16).

**18.** Structure absorbante (1) selon la revendication 16, **caractérisée en ce que** la première et la deuxième couche (3, 4) absorbante externe sont identiques en ce qui concerne la valeur de pH des premières et des deuxièmes fibres de cellulose (6, 16) et en ce qui concerne la proportion pondérale de premières et de deuxièmes fibres de cellulose (6, 16).

**19.** Structure absorbante (1) selon la revendication 16, **caractérisée en ce que** la première et la deuxième couche (3, 4) absorbante externe sont identiques en ce qui concerne la valeur de pH des premières et des deuxièmes fibres de cellulose (6, 16) et en ce qui concerne la composition chimique ou la provenance naturelle ou l'origine des premières et de deuxièmes fibres de cellulose.

**20.** Structure absorbante (1) selon la revendication 16, **caractérisée en ce que** la première et la deuxième couche (3, 4) absorbante externe sont identiques en ce qui concerne la valeur de pH des premières et des deuxièmes fibres de cellulose (6, 16), en ce qui concerne la composition chimique ou la provenance naturelle ou l'origine des premières et des deuxièmes fibres de cellulose et en ce qui concerne la proportion pondérale de premières et de deuxièmes fibres de cellulose (6, 16).

**21.** Structure absorbante (1) selon la revendication 16, **caractérisée en ce que** la première et la deuxième couche (3, 4) absorbante externe sont identiques en ce qui concerne toutes les propriétés.

**22.** Structure absorbante (1) selon l'une quelconque des revendications précédentes, **caractérisée en ce que** la succession (2) de couches reliées les unes aux autres présente une symétrie par rapport à un miroir.

**23.** Structure absorbante (1) selon l'une quelconque des revendications précédentes, **caractérisée en ce que** la structure absorbante (1) forme un article absorbant (20) ou est contenue dans un article absorbant (20).

**24.** Article absorbant (20) comprenant une structure absorbante (1) selon l'une quelconque des revendications précédentes 1 à 22.

**25.** Article absorbant (20) selon la revendication 24, **caractérisé en ce que** l'article absorbant est une protection pour incontinence, une serviette hygiénique, une couche pour incontinence de type ouvert pourvue de systèmes de fermeture, une couche pour incontinence de type fermé et/ou un pansement.

**Fig. 1**

**Fig. 2**

**Fig. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102009055951 A1
- DE 102010006228 A1
- EP 1191915 B1
- EP 0138179 A2
- EP 0202127 B1
- EP 0991436 B1
- EP 2086596 B1
- WO 2008138386 A1
- EP 0832320 B1
- US 6852904 B2